# NEUE EUROPÄISCHE PATENTSCHRIFT

(11) **EP 1 847 258 B2**
(45) Veröffentlichungstag und Bekanntmachung des Hinweises auf die Entscheidung über den Einspruch: **16.01.2013**
(45) Hinweis auf die Patenterteilung: 21.04.2010
(21) Anmeldenummer: 06007841.7
(22) Anmeldetag: 13.04.2006
(51) Int. Cl.: A61K 9/20

(54) **Partialglyceride als Schmiermittel für pharmazeutische Zusammensetzungen enthaltend Thieno[3,2-c]pyridin-Derivate**
Partial glycerides as lubricants in pharmaceutical compositions comprising thieno[3,2-c]pyridine derivatives
Compositions pharmaceutiques comprenant thieno[3,2-c]pyridine comme agent active et des glycérols partielles comme lubrifiante

(43) Veröffentlichungstag der Anmeldung: 24.10.2007
(73) Patentinhaber: Acino Pharma AG, 4253 Liesberg (CH)
(72) Erfinder: Beckert, Thomas Dr., 88447 Warthausen-Birkenhard (DE); Braun, Michael, 89250 Senden (DE); Neuer, Klaus, 88477 Schwendi-Orsenhausen (DE)
(74) Vertreter: Westendorp | Sommer

(56) Entgegenhaltungen:
- EP-A- 0 368 766
- EP-A- 1 005 864
- WO-A-2005/048991
- WO-A1-2004/100932
- WO-A1-2007/091279
- US-A- 4 490 377
- US-A- 5 872 128
- "Handbook of Pharmaceutical Excipients", 5th Ed., S.304-305, Monographie "Glyceryl Behenate"
- Drug Development Industrial Pharmacy, 12(8&9), S. 1329-1346(1986)
- Enzyklopädie-Artikel "Glycerinacetate", Römpp Chemielexikon, S.1610, 1995
- Enzyklopädie-Artikel "Glycerinester" und "Glyceride", Römpp Chemielexikon, S.1609, 1611, 1995
- Artikel "GLYCEROL DIBEHENATE" der European Pharmacopoeia 4
- Artikel "synonym" und "Synonym", Richtig Schreiben, Deutsche Rechtschreibung & Fremdwörterlexikon, 2006, S. 724

## Beschreibung

Die Erfindung betrifft stabile Formulierungen enthaltend Thieno[3,2-c]pyridin-Derivate als Wirkstoff, im besonderen Clopidogrelhydrogensulfat oder andere pharmazeutisch annehmbare Salze.

Thieno[3,2-c]pyridin-Derivate sind eine bekannte Wirkstoffklasse, die insbesondere als Thrombozyten-Aggregationshemmer eingesetzt werden, zu der unter anderem Clopidogrel und Ticlopidin zählen. Diese Wirkstoffe werden unter anderem in Tabletten formuliert, wobei geeignete Schmier- und Formentrennmittel nötig sind. Schmier- und Formentrennmittel sind Hilfsstoffe, die die interpartikuläre Reibung während der Kompression reduzieren und die Reibung zwischen Tablette und Matrizenwand während des Ausstoßens herabsetzen sowie ein Kleben an den Stempelwerkzeugen der Tablettenmaschine verhindern sollen, teilweise auch in Füllmassen für HartgelatineKapseln, wenn bei der Abfüllung eine Komprimierung erfolgt.

Schnell freisetzende pharmazeutische Tablettenformulierungen sollen neben einer hohen Zerfallsgeschwindigkeit im Allgemeinen auch eine ausreichend hohe Bruchfestigkeit aufweisen. Eine entsprechende mechanische Stabilität gewährleistet eine unkomplizierte Weiterverarbeitung zum Beispiel bei der Konfektionierung und trägt zur Arzneimittelsicherheit bei. Beide Eigenschaften werden, zum Teil in entscheidender Weise, durch das verwendete Schmier- und Formentrennmittel beeinflusst.

Thieno[3,2-c]pyridin-Derivate sind bekannt dafür, gegenüber zahlreichen gängigen pharmazeutischen Hilfsstoffen wie Gelatine oder Povidon empfindlich zu reagieren, d.h. abgebaut zu werden.

Vor allem das basisch reagierende Standardschmiermittel Magnesiumstearat bewirkt signifikanten Wirkstoffabbau in der Tablettenformulierung. Dieses Problem ist bekannt und wird zum Beispiel in US 4,591,592 beschrieben. Das Problem kann durch Zusätze von organischen Säuren minimiert werden (siehe zum Beispiel US 4,591,592), dies gestaltet jedoch die Formulierungen unerwünscht komplex. Weiterhin wurden verschiedene Alternativen für das Standardschmiermittel Magnesiumstearat vorgeschlagen, um einen Wirkstoffabbau zu verhindern und so die Stabilität der Formulierung zu gewährleisten.

US 5,520,928 betrifft die Stabilisierung einer pharmazeutischen Zusammensetzung, die das Thieno[3,2-c]pyridin-Derivat Ticlopidinhydrochlorid enthält. Die Stabilisierung wird durch die Verwendung von Stearinsäure erreicht, die auch als Schmiermittel dient. Des Weiteren umfasst die pharmazeutische Zusammensetzung gemäß US 5,520,928 mindestens einen Hilfsstoff ausgewählt aus Desintegrationsmittel, Bindemittel und Verdünner, die im Wesentlichen frei von organischen Säuren sind, mit Ausnahme von Stearinsäure.

WO 2005/070464 betrifft eine stabile pharmazeutische Zusammensetzung enthaltend als Wirkstoff Clopidogrelhydrogensulfat. Als Schmiermittel werden verschiedene Alternativen zum Standardschmiermittel Magnesiumstearat offenbart. Neben Natriumcarboxylmethylstärke wird hydriertes Pflanzenöl als Schmiermittel offenbart.

EP 1 310 245 betrifft ebenfalls pharmazeutische Zusammensetzungen in der Form von Tabletten, umfassend Clopidogrelhydrogensulfat sowie ein Schmiermittel. Um die Stabilität der Zusammensetzung zu erhöhen, werden als Alternative zu den Schmiermitteln Magnesiumstearat und Calciumstearat die Schmiermittel Zinkstearat, Stearinsäure und Natriumstearylfumarat vorgeschlagen.

WO 00/01364 betrifft eine stabile pharmazeutische Zusammensetzung umfassend Thieno[3,2-c]pyridin-Derivate sowie als wasserlösliches hydrophiles Schmiermittel Polyethylenglykol und mindestens einen pharmazeutisch annehmbaren Hilfsstoff. Ein bevorzugtes Thieno[3,2-c]pyridin-Derivat ist Ticlopidin oder Ticlopidinhydrochlorid. Die pharmazeutische Zusammensetzung ist im Wesentlichen frei von Magnesiumstearat, wasserlöslichem Polyvinylpyrrolidon und Natriumstärkeglycolat.

WO 2005048992 offenbart einen weiteren Lösungsansatz, in dem mit Polyvinylacetat oder Polyvinylalkoholat überzogene Wirkstoffpartikel, -granulate oder -agglomerate eingesetzt werden. Um eine erhöhte Stabilität des Wirkstoffs zu erhalten, wird als Schmiermittel hydriertes Pflanzenöl vorgeschlagen. Statt Hydrogensulfat werden andere pharmazeutisch geeignete Salze, wie das Mesilat, Hydrobromid oder Hydrochlorid eingesetzt. Als weitere hydrophobe Komponenten können u.a. Partialglyceride enthalten sein.

WO 2004098593 betrifft eine stabile, nicht-hygroskopische pharmazeutische Zusammensetzung, die amorphes Clopidogrel enthält. Weiterhin umfasst die Zusammensetzung Calcium- oder Magnesiumstearat sowie einen nicht-hygroskopischen Zusatzstoff mit mindestens einem weiteren Hilfsstoff.

Vor diesem Hintergrund wurde ein neues effektives Schmiermittel gesucht, mit dessen Hilfe auf einfachem Weg stabile pharmazeutische Zusammensetzungen enthaltend Thieno[3,2-c]pyridin-Derivate hergestellt werden können, wobei Vorteile wie z.B. eine erhöhte Zerfallsgeschwindigkeit bei einer schnell freisetzenden pharmazeutischen Tablettenformulierung und eine erhöhte Bruchfestigkeit erhalten werden.

Aufgabe der Erfindung ist es, ein Schmiermittel für eine pharmazeutische Zusammensetzung enthaltend ein Thieno[3,2-c]pyridin-Derivat bereitzustellen, so dass sich ein schneller Zerfall der Zusammensetzung und/oder eine erhöhte Bruchfestigkeit zeigt.

Vorstehende Aufgabe wird durch die Gegenstände der unabhängigen Ansprüche 1, und 9 - 11 gelöst. Vorteilhafte Weiterentwicklungen gehen aus den Unteransprüchen hervor.

Vorteilhaft wird gemäß der Erfindung ein neues Schmiermittel eingesetzt. Es werden Partialglyceride, wie insbesondere Glycerolbehenat oder Glycerolpalmitostearat als Schmiermittel verwendet.

Vorteilhaft enthält eine pharmazeutische Zusammensetzung:
ein Thieno[3,2-c]pyridin-Derivat als Wirkstoff und
ein Partialglycerid.

Das Partialglycerid kann hierbei einen Schmelzpunkt im Bereich von 45 °C bis 85°C aufweist, bevorzugter Weise im Bereich von 69 - 74°C oder 53 - 57°C.

Das Partialglycerid ist in der pharmazeutischen Zusammensetzung in einer Menge von 0,1 - 25 Gew.%, bevorzugt 6 - 17 Gew.%, stärker bevorzugt 10 - 15 Gew.% und am stärksten bevorzugt 12 - 13 Gew.%, bezogen auf das Gesamtgewicht der Pharmazeutischen Zusammensetzung enthalten.

Bevorzugte Untergrenzen für den Gehalt des Partialglycerids in der pharmazeutischen Zusammensetzung sind 0,1, 6, 10 und 12 Gew.%. Bevorzugte Obergrenzen für den Gehalt des Partialglycerids in der pharmazeutischen Zusammensetzung sind 25, 17, 15, 13 Gew.%.

Das Partialglycerid ist bevorzugter Weise Glycerolbehenat oder Glycerolpalmitostearat. Die pharmazeutische Zusammensetzung kann als Tablette, Kapsel, Mikrotablette, Nanotablette, Granulat, Pulver als in Kapseln gefüllte Mikrotabletten, Nanotabletten, Granulat und Pulver oder als in Sacchets gefüllte(s) Mikrotabletten, Nanotabletten, Granulat und Pulver vorliegen, jedoch vorzugsweise als Tablette.

Weiterhin betrifft die Erfindung die Verwendung eines Schmiermittels, ausgewählt aus der Gruppe der Partialglyceride zur Herstellung einer pharmazeutischen Zusammensetzung, wobei die pharmazeutische Zusammensetzung ein Thieno[3,2-c]pyridin-Derivat enthält.

Die Erfindung betrifft auch ein Verfahren zur Herstellung einer pharmazeutischen Zusammensetzung, enthaltend ein Thieno[3,2-c]pyridin-Derivat, bei der als Schmier- und Formentrennmittel ein Partialglycerid verwendet wird.

Das Thieno[3,2-c]pyridin-Derivat (im Folgenden auch Wirkstoff bezeichnet) kann als freie Base vorliegen oder als ein beliebiges pharmazeutisch annehmbares Salz davon. Sofern der Wirkstoff als Salz vorliegt, ist das Anion bevorzugter Weise ausgewählt aus der Gruppe, bestehend aus Sulfat, Hydrogensulfat, Bromid, Chlorid, Iodid, Mesilat, Carbonat und Hydrogencarbonat, bevorzugter Weise aus Chlorid, Hydrogensulfat und Mesilat. Das Thieno[3,2-c]pyridin-Derivat ist vorzugsweise ausgewählt aus der Gruppe, bestehend aus Clopidogrel und Ticlopidin sowie Mischungen davon.

Die Aufgabe der Erfindung wird durch den Einsatz von Partialglyceriden als Schmiermittel gelöst. Darunter werden die Mono- und Diester von Glycerin (Glycerol) und Fettsäuren (bzw. Carbonsäuren) verstanden oder anders ausgedrückt eine Glycerineinheit ist entweder mit einer Fettsäure-Einheit (Monoglycerid) oder zwei Fettsäure-Einheiten (Diglycerid) verestert. Das Partialglycerid kann ein Mono- oder Diglycerid sein.

Die Fettsäure des Partialglycerids ist bevorzugter Weise ausgewählt aus der Gruppe bestehend aus Hexansäure, Heptansäure, Caprylsäure, Pelargonsäure, Decansäure, Undecansäure, Laurinsäure, Tridecansäure, Tetradecansäure, Pentadecansäure, Palmitinsäure, Margarinsäure, Stearinsäure, Nonadecansäure, Arachinsäure, Behensäure, Lignocerinsäure und Melissinsäure sowie Mischungen davon. Bevorzugter Weise ist die Fettsäure Behensäure, Palmitinsäure oder Stearinsäure. Bei dem Partialglycerid handelte es sich bevorzugter Weise um Glycerolbehenat oder Glycerolpalmitostearat. Besonders bevorzugt ist Glycerolbehenat wie es z.B. unter dem Handelsnahmen Compritol ATO 888 ® im Handel erhältlich ist.

Das Partialglycerid ist in der pharmazeutischen Zusammensetzung in einer Menge von 0,1 bis 25 Gew.%, bevorzugt 6 bis 17 Gew.%, stärker bevorzugt 10 bis 15 Gew.% und am stärksten bevorzugt 12 bis 13 Gew.%, bezogen auf das Gesamtgewicht der pharmazeutischen Zusammensetzung, enthalten.

Bevorzugt ist, dass das Partialglycerid unter Raumbedingungen (d.h. SATP, entsprechend 100 kPa, und 25°C) fest ist.

Das Partialglycerid liegt möglicherweise in einem Gemisch verschiedener Partialglyceride vor und ist darin in über 50 Gew.%, über 70 Gew.% und bevorzugter Weise über 90 Gew.%, bezogen auf die Gesamtmenge des Gemisches der verschiedenen Partialglyceride anwesend.

Die Schmier- und Formentrennmittel der vorliegenden Erfindung weisen neben Schmiermitteleigenschaften und Trennmitteleigenschaften auch Bindemitteleigenschaften auf.

Die Schmier- Trenn- und Bindemitteleigenschaften der Partialglyceride der vorliegenden Erfindung wirken sich positiv auf die Prozessfähigkeit aus, da auf Hochleistungstablettenpressen weniger Tabletten zum Deckeln neigen.

Zum anderen lassen sich Bruchfestigkeit und Friabilität der Komprimate verbessern, ohne die Zerfallszeit negativ zu beeinflussen. Dies ist im vorliegenden Falle besonders wichtig, daThieno[3,2-c]pyridin-Derivate wie Clopidogrel enthaltende Tablettenkerne bevorzugt mit Isolierfilmen wie z.B. Opadry^{®}AMB oder EUDRAGIT^{®} E PO überzogen werden.

Die pharmazeutische Zusammensetzung ist bevorzugter Weise eine schnell freisetzende pharmazeutische Formulierung. Diese kann neben dem Wirkstoff einen oder mehrere Füll- und Bindemittel, Zerfallhilfsmittel, Schmier- und Formentrennmittel sowie Fließregulierungsmittel enthalten. Es kann zum Beispiel mikrokristalline Zellulose als gängiger Füllstoff mit sehr guter plastischer Verformbarkeit zusammen mit dem Trockenbindemittel Copovidon eingesetzt werden, wobei gute Bruchfestigkeits- und Abriebswerte erzielt werden. Mannitol als löslicher Zuckeralkohol gewährleistet zusammen mit dem effektiven Zerfallsförderer Crospovidon ein schnelles Quellen und Auflösen der Tablette, um den Wirkstoff rasch zu liberieren. Es können aber auch andere Füll- und Bindemittel oder Zerfallhilfsmittel mit vergleichbaren Eigenschaften eingesetzt werden.

Das Schmier- und Formentrennmittel der Erfindung ermöglicht es, auf einfachem Weg stabile Tablettenzubereitungen mit Thieno[3,2c]pyridin-Derivaten herzustellen, wobei die bei hydrophoben Schmiermitteln oft beobachteten Nachteile, wie verlangsamter Tablettenzerfall und/oder reduzierte Tablettenbruchfestigkeit weniger stark ausgeprägt sind. Überraschenderweise konnte festgestellt werden, dass Tabletten mit einem Partialglycerid als Schmiermittel viel stabiler sind als Tabletten mit äquivalentem Zusatz an Magnesiumstearat. Der Wirkstoffabbau verläuft etwa gleich langsam wie bei Tabletten mit hydriertem Pflanzenöl als Schmier- und Formentrennmittel, jedoch werden die genannten Nachteile vermieden. Gegenüber Magnesiumstearat und hydriertem Pflanzenöl zeigten die Tablettenkerne eine für die spätere Befilmung erwünschte höhere Bruch- und Abriebfestigkeit, ohne dass es dabei zu einer erhöhten Degradation (d.h. Abbau) des Wirkstoffs gekommen wäre. Diese vorteilhaften Eigenschaften der Schmiermittel der Erfindung werden in den Beispielen demonstriert.

Im Folgenden erfolgt eine detaillierte Beschreibung der Erfindung anhand von Beispielen. Dabei werden weitere Merkmale der Erfindung offenbart, die untereinander kombiniert werden können.

### Beispiele

### Beispiel 1

111,8 g Clopidogrelhydrogensulfat,
41,2 g Mannitol DC,
79,9 g MCC 102,
37,1 g MCC 200,
4,3 g Copovidon,
10,3 g Crospovidon,
14,3 g Glycerolbehenat,
1,1 g hochdisperses Silicuiumdioxid
werden in einem Laborfreifallmischer gemischt und anschließend zu runden Tabletten mit einem Durchmesser von 9 mm verpresst.

### Beispiel 2

Wie Beispiel 1, nur dass anstatt Glycerolbehenat Magnesiumstearat verwendet wurde.

### Beispiel 3

Wie Beispiel 1, nur dass anstatt Glycerolbehenat hydriertes Pflanzenöl verwendet wurde.

Tabelle 1 gibt einen Überblick über die Zusammensetzungen der nach Beispielen 1 - 3 hergestellten Tabletten.

**Tabelle 1**

| **Komponente** | **Menge [mg/DF]** | **Menge [mg/DF]** | **Menge [mg/DF]** | **Funktion** |
|---|---|---|---|---|
| | Bsp 1 | Bsp 2 | Bsp 3 | |
| Clopidogrelhydrogensulfa | 111,8 | 111,8 | 118,8 | Aktive Komponente |
| Mannitol DC | 41,2 | 41,2 | 41,2 | Füllstoff/Bindemittel |
| MCC 102 | 79,9 | 79,9 | 79,9 | Füllstoff/Bindemittel |
| MCC 200 | 37,1 | 37,1 | 37,1 | Füllstoff/Bindemittel |
| Copovidon | 4,3 | 4,3 | 4,3 | Trockenbindemittel |
| Crospovidon | 10,3 | 10,3 | 10,3 | Zerfallsförderer |
| Glycerolbehenat | 14,3 | | | Schmier- u. Trennmittel |
| Magnesiumstearat pfl. | | 14,3 | | Schmier- u. Trennmittel |
| Hydriertes Pflanzenöl | | | 14,3 | Schmier- u. Trennmittel |
| Siliciumdioxid, hochdisp. | 1,1 | 1,1 | 1,1 | Fliessregulierungsmittel |
| Summe | 240 | 240 | 240 | |

### Physikalische Charakterisierung

Die diametrale Bruchfestigkeit und die Zerfallszeit der Tabletten wurde in Übereinstimmung mit den entsprechenden Apparaturen und Methoden des Europäischen Arzeinbuchs bestimmt.

Tabelle 2 zeigt die Bruchfestigkeit und die Zerfallsgeschwindigkeit der in Beispiel 1 - 3 hergestellten Tablettenformulierungen. Die Ergebnisse zeigen, dass durch die Schmiermittel der Erfindung deutlich erhöhte Bruchfestigkeiten bei vergleichbaren Zerfallsgeschwindigkeiten erhalten werden.

**Tabelle 2**

| **Eigenschaft** | **Beispiel 1** | **Beispiel 2** | **Beispiel 3** |
|---|---|---|---|
| Bruchfestigkeit [N] (n=10) | ~120 | ~105 | ~80 |
| Zerfall [min] | 5 bis 7 | 5 bis 7 | 5 bis 7 |

### Stabilitätsergebnisse

Die in den Beispielen 1 - 3 hergestellten Tabletten wurden in PVC-Alu-Folie eingeblistert und bei 60°C in einem Klimaschrank eingelagert. Nach jeweils zwei und vier Wochen wurden entsprechende Proben mittels einer geeigneten HPLC-Methode untersucht. Zu Vergleichszwecken wurde der unter gleichen Bedingungen eingelagerte reine Wirkstoff zu den entsprechenden Prüfzeitpunkten ebenfalls mit untersucht.

Die entstandenen, aufsummierten Abbauprodukte werden in Tabelle 3 in Prozent, bezogen auf den initialen Wirkstoffgehalt angegeben.

**Tabelle 3**

| **Untersuchungszeitpunkt** | **Clopidogrelhydrogensulfat reiner Wirkstoff** | **Formulierung nach Beispiel 1** | **Forumulierung nach Beispiel 2** | **Formulierumg nach Beispiel 3** |
|---|---|---|---|---|
| Ausgangswert | 0,92% | 1,25% | 1,20% | 1,18% |
| 2 Wochen | 0,99% | 1,50% | 3,10% | 1,44% |
| 4 Wochen | 1,19% | 1,66% | 4,19% | 1,65% |

Aus der Tabelle geht eindeutig hervor, dass bei Verwendung von Glycerolbehenat als Schmiermittel ein gegenüber Magnesiumstearat deutlich besseres und gegenüber hydriertem Pflanzenöl ein vergleichbares Ergebnis erzielt werden kann.

## Patentansprüche

1. Pharmazeutische Zusammensetzung enthaltend:
a. ein Thieno[3,2-c]pyridin-Derivat als Wirkstoff und
b. ein Partialglycerid,
wobei das Partialglycerid in einer Menge von 0,1 - 25 Gew.%, bezogen auf das Gesamtgewicht der pharmazeutischen Zusammensetzung, enthalten ist.

2. Pharmazeutische Zusammensetzung nach Anspruch 1, wobei das Partialglycerid einen Schmelzpunkt im Bereich von 45°C bis 85°C aufweist, bevorzugter Weise im Bereich von 69 - 74 °C oder 53 - 57 °C.

3. Pharmazeutische Zusammensetzung nach Anspruch 1, wobei das Partialglycerid ein festes Partialglycerid ist.

4. Pharmazeutische Zusammensetzung nach Anspruch 1, wobei das Partialglycerid in einem Gemisch verschiedener Partialglyceride vorliegt und darin in über 50 Gew.%, über 70 Gew.% und bevorzugter Weise über 90 Gew.% anwesend ist.

5. Pharmazeutische Zusammensetzung nach Anspruch 1, wobei das Partialglyceride ein Mono- oder Diglycerid ist.

6. Pharmazeutische Zusammensetzung nach Anspruch 1, wobei das Partialglycerid in einer Menge von bevorzugt 6 - 17 Gew.%, stärker bevorzugt 10 - 15 Gew.% und am stärksten bevorzugt 12 - 13 Gew.%, bezogen auf das Gesamtgewicht der pharmazeutischen Zusammensetzung, enthalten ist.

7. Pharmazeutische Zusammensetzung nach Anspruch 1, wobei das Partialglycerid ausgewählt ist aus der Gruppe bestehend aus Glycerolbehenat und Glycerolpalmitostearat sowie Mischungen davon.

8. Pharmazeutische Zusammensetzung nach Anspruch 1, wobei die pharmazeutische Zusammensetzung als Tablette, Kapsel, Mikrotablette, Nanotablette, Granulat, Pulver oder als in Kapseln gefüllte(s) Mikrotabletten, Nanotabletten, Granulat und Pulver oder als in Sacchets gefüllte(s) Mikrotabletten, Nanotabletten, Granulat und Pulver vorliegt, jedoch vorzugsweise als Tablette vorliegt.

9. Verwendung eines Partialglycerids als Schmiermittel, zur Herstellung einer pharmazeutischen Zusammensetzung, enthaltend mindestens ein Thieno-[3,2-c]pyridin-Derivat,
wobei das Partialglycerid in einer Menge von 0,1 - 25 Gew.%, gezogen auf das Gesamtgewicht der phannazeutischen Zusammensetzung. in derpharmazeutischen Zusammensetzung enthalten ist.

10. Verwendung eines Schmiermittels, ausgewählt aus der Gruppe der Partialglyceride zur Herstellung einer pharmazeutischen Zusammensetzung nach Anspruch 1.

11. Verfahren zur Herstellung einer pharmazeutischen Zusammensetzung, enthaltend ein Thieno[3,2-c]pyridin-Derivat, bei dem als Schmiermittel ein Partialglycerid verwendet wird,
wobei das Partial glycerid in einer Menge von 0.1 - 25 Gew.%, bezogen auf das Gesamtgewicht der pharmazeutischen Zusammensetzung, in derpharmazeutischen Zusammensetzung enthalten ist.

## Claims

1. A pharmaceutical composition, comprising:
a. a derivative of thieno[3,2-c]pyridine as an active ingredient and
b. a partial glyceride,
wherein the partial glyceride is contained in an amount of 0.1 - 25 % by weight, based on the total weight of the pharmaceutical composition.

2. The pharmaceutical composition according to claim 1, wherein the partial glyceride has a melting point in the range of 45°C to 85°C, preferably in the range of 69 - 74°C or 53 - 57°C.

3. The pharmaceutical composition according to claim 1, wherein the partial glyceride is a solid partial glyceride.

4. The pharmaceutical composition according to claim 1, wherein the partial glyceride is present in a mixture of different partial glycerides and is present therein in an amount of more than 50 % by weight, more than 70 % by weight and preferably more than 90 % by weight.

5. The pharmaceutical composition according to claim 1, wherein the partial glyceride is a mono- or diglyceride.

6. The pharmaceutical composition according to claim 1, wherein the partial glyceride is contained in an amount of preferably 6 - 17 % by weight, more preferably 10 - 15 % by weight and most preferably 12 - 13 % by weight, based on the total weight of the pharmaceutical composition.

7. The pharmaceutical composition according to claim 1, wherein the partial glyceride is selected from the group consisting of glycerol behenate and glycerol palmito stearate as well as mixtures thereof.

8. The pharmaceutical composition according to claim 1, wherein the pharmaceutical composition is a tablet, capsule, micro tablet, nano tablet, granulate, powder; or micro tablets, nano tablets, granulate and powder filled in capsules; or micro tablets, nano tablets, granulate and powder filled in sachets, however preferably is a tablet.

9. Use of a partial glyceride as a lubricant for the production of a pharmaceutical composition, comprising at least one derivative of thieno[3,2-c]pyridine, wherein the partial glyceride is contained in the pharmaceutical composition in an amount of 0.1 - 25 % by weight, based on the total weight of the pharmaceutical composition.

10. Use of a lubricant, selected from the group of partial glycerides, for the production of a pharmaceutical composition according to claim 1.

11. A method for the production of a pharmaceutical composition, comprising a derivate of thieno[3,2-c]pyridine, wherein a partial glyceride is used as a lubricant, wherein the partial glyceride is contained in the pharmaceutical composition in an amount of 0.1 - 25 % by weight, based on the total weight of the pharmaceutical composition.

## Revendications

1. Composition pharmaceutique comprenant:
a. un dérivé de thieno[3,2-c]pyridine comme substance actif et
b. un glycéride partiel,
dans laquelle le glycéride partiel est contenu dans une quantité de 0,1 - 25 % en poids par rapport au poids total de la composition pharmaceutique.

2. Composition pharmaceutique selon la revendication 1, dans laquelle le glycéride partiel a un point de fusion dans la gamme de 45 °C à 85 °C, préférentiellement dans la gamme de 69-74 °C ou 53-57 °C.

3. Composition pharmaceutique selon la revendication 1, dans laquelle le glycéride partiel est un glycéride partiel solide.

4. Composition pharmaceutique selon la revendication 1, dans laquelle le glycéride partiel se trouve dans un mélange de divers glycérides partiels et y est présent en une teneur supérieure à 50 % en poids, supérieure à 70 % en poids et préférentiellement supérieure à 90 % en poids.

5. Composition pharmaceutique selon la revendication 1, dans laquelle le glycéride partiel est un monoglycéride ou diglycéride.

6. Composition pharmaceutique selon la revendication 1, dans laquelle le glycéride partiel est contenu dans une quantité préférentiellement de 6-17 % en poids, plus préférentiellement de 10-15 % en poids et très préférentiellement de 12-13 % en poids par rapport au poids total de la composition pharmaceutique.

7. Composition pharmaceutique selon la revendication 1, dans laquelle le glycéride partiel est choisi parmi le group consistant en le béhénate de glycérol et le palmitostearate de glycérol ainsi que les mélanges de ceux-ci.

8. Composition pharmaceutique selon la revendication 1, dans laquelle la composition pharmaceutique est présente sous forme d'un comprimé, gélule, microcomprimé, nanocomprimé, des granules, d'un poudre ou sous forme d'un (des) comprimé(s), d'un (des) microcomprimé(s), des granules et d'un poudre rempli(s) dans des gélules ou sous forme d'un (des) comprimé(s), d'un (des) microcomprimé(s), des granules et d'un poudre rempli(s) dans des sachets, mais préférentiellement sous forme d'un comprimé.

9. Utilisation d'un glycéride partiel comme lubrifiant pour la production d'une composition pharmaceutique comprenant au moins un dérivé de thieno[3,2-c]pyridine dans laquelle le glycéride partiel est contenu dans une quantité de 0,1 - 25 % en poids par rapport au poids total de la composition pharmaceutique.

10. Utilisation d'un lubrifiant choisi parmi le group des glycérides partiels pour la production d'une composition pharmaceutique selon la revendication 1.

11. Procédé pour la production d'une composition pharmaceutique comprenant un dérivé de thieno[3,2-c]pyridine dans lequel un glycéride partiel est utilisé comme lubrifiant, dans lequel le glycéride partiel est contenu dans une quantité de 0,1 - 25 % en poids par rapport au poids total de la composition pharmaceutique.
